Europäisches Patentamt

**European Patent Office**

Office européen des brevets

(19)

(11) Publication number: **0 257 565 B1**

(12)

# EUROPEAN PATENT SPECIFICATION

(45) Date of publication of patent specification:
09.01.91 Bulletin 91/02

(51) Int. Cl.⁵: **C07C 57/04,** C07C 51/25, C07C 45/35, C07C 47/22

(21) Application number: 87112102.6

(22) Date of filing: 20.08.87

(54) **Anhydrous diluent process for the propylene oxidation reaction to acrolein and acrolein oxidation to acrylic acid.**

(30) Priority: 21.08.86 US 898491

(43) Date of publication of application:
02.03.88 Bulletin 88/09

(45) Publication of the grant of the patent:
09.01.91 Bulletin 91/02

(84) Designated Contracting States:
AT BE CH DE ES FR GB GR IT LI LU NL SE

(56) References cited:
DE-A- 2 202 734
GB-A- 2 044 764
US-A- 3 736 355
US-A- 4 031 135
US-A- 4 124 634
US-A- 4 442 308

(73) Proprietor: UNION CARBIDE CORPORATION
39 Old Ridgebury Road
Danbury Connecticut 06817 (US)

(72) Inventor: Etzkorn, William George
5001 Lee Drive
Cross Lanes West Virginia 25313 (US)
Inventor: Harkreader, Gordon Gene
1506 Brentwood Road
Charleston West Virginia 25314 (US)

(74) Representative: Weinhold, Peter, Dr. et al
Patentanwälte Dr. V. Schmied-Kowarzik
Dipl.-Ing. G. Dannenberg Dr. P. Weinhold Dr. D.
Gudel Dipl.-Ing. S. Schubert Dr. P. Barz
Siegfriedstrasse 8
D-8000 München 40 (DE)

## Description

## BACKGROUND OF THE INVENTION

Field of the Invention

This invention relates to a process for the manufacture of acrolein or acrylic acid from propylene. More specifically, it describes an improved process for producing acrolein or producing acrylic acid by the catalytic vapor phase oxidation of propylene in the presence of inert, anhydrous diluents.

Summary of the Prior Art

Generally, propylene in its gaseous phase is oxidized to acrolein in the presence of molecular oxygen-containing gases and steam by contact at elevated temperatures with solid metal oxide catalysts. The acrolein produced in this reaction stage can be recovered or can be directed without separation of the acrolein to a second reactor operating in series with the first reactor to oxidize the acrolein to acrylic acid.

In the prior art, steam has been used in the starting reactant gas mixture in order to avoid flammable gas mixtures and because it was believed to be important to reaction selectivity. For example, US Patent No. 4,147,885 relates that it is wide practice to incorporate steam to avoid burning the reactant gases and increase selectivity to acrylic acid. US Patent No. 3,475,488 discloses that it is desirable to incorporate steam in the starting reactant gas since this increases conversion and selectivity when employed in the order of 1 to 60, and preferably 5 to 30, moles of steam per mole of propylene or propylene + acrolein.

Other patents also describe steam as the preferred diluent. For example, US Patent 3,171,859 states that "addition of steam is obligatory ... it acts not only as a diluent, but also favors the reaction in that combustion to carbon oxides is substantially reduced." Also, US Patent 4,267,386 reiterates the general understanding among those skilled in the art, that while inert diluents may be added to the reaction system, "water, in the form of steam is desirably present ... in amounts of from 0.5 to 15, preferably 2 to 15, moles per mole of unsaturated hydrocarbon (i.e., propylene or acrolein)."

Many oxidation catalysts have been disclosed for producing acrolein in high yield by oxidizing propylene. Predominantly, these are catalysts containing mixed oxides of molybdenum, bismuth and iron with phosphorous or tungsten or antimony. Cobalt and/or nickel and alkali metals are common promoters.

Catalysts which have been found to be advantageous for use in oxidizing acrolein to acrylic acid at conversions of more than 98% generally contain mixed metal oxides. Such catalysts typically contain molybdenum, vanadium, tungsten, chromium, copper, niobium, tantalum and antimony.

The ultimate object of the teachings in the literature cited above is to obtain high performance catalysts which give high selectivities to acrolein and acrylic acid at high propylene conversions. Other factors which influence the economic viability or the improved performance of these processes are not considered in these prior art techniques. For example, they do not address the impact on process variables of use of high propylene concentrations, how to avoid the danger of explosion, the impact of inert reaction process feeds on recovery and waste disposal, or maintaining high catalyst performance over an extended catalyst life. These are all extremely important for commercial operation.

In commercial operation, it is of economic importance to minimize the presence of the steam which is fed to the reactors, since it passes through the system and becomes a burdensome wastewater load after product recovery steps ; nevertheless, to the knowledge of the present inventors, no commercial process has been successfully operated below a steam : propylene mole ratio of about 1.5. Furthermore, it is extremely important to minimize by-products which are difficult to separate from useful product or which carry a high economic penalty for disposal. Process improvements which will provide high catalyst performance while simultaneously maximizing propylene feedstock usage, and improvements which may promote conditions for extended useful catalyst life are important for commercial operation. The prior art does not adequately address these issues.

US Patent 4,049,577 teaches an improved catalyst composition for making acrolein. The authors mention that recycle gas comprised of the noncondensable fraction of the product can be used in place of steam. They suggest that these recycled inerts are preferable to steam as diluent since they allow higher conversions of propylene and thus enable one to obtain higher yields, and also reduce the water load on the system ; however, the use of recycled inerts is stated as being made possible by the characteristics of this particular catalyst composition. Nowhere does this patent suggest or teach that anhydrous diluents have an improved effect on selectivity or product mix, or are useful with other catalysts. The relationships between various diluents and the heat capacity effects on selectivity are not suggested.

US Patent 3,801,634 teaches the use of inert solids mixed with active catalysts in the first and second stage reactors used to manufacture acrolein and acrylic acid. The authors indicate that noncondensable, second-stage effluent gases can be recycled to the first stage as inert diluting gas which can, at least partly, replace steam. The authors do not show any relationship between the inert anhydrous diluent gases and improvements in product selectivity.

US Patent 4,031,135 presents a recycle process in which noncondensable gases, preferably including steam, are recycled to the first-stage reactor and also to the interstage (second-stage) reactor feed. There is no recognition of the benefits in using anhydrous diluents with respect to their effect on by-product selectivity mix. The authors do, however, recognize an apparent improved acrylic acid efficiency, which they attribute partly to the use of inert diluents other than steam. They do not prove that this is true, nor do they show the effect of heat capacity on product selectivities.

US Patent 4,365,087 refers to the recycling of dewatered residue gas, containing both inert and reactive gases, to increase the concentration of acrylic acid recovered. However, the authors consider this procedure unsatisfactory since the composition of the residue gas fluctuates.

US Patent 4,442,308 teaches the use of inert gases as diluent in the acrolein process ; however, it specifies their use for a particular supported first-stage acrolein catalyst. Most common commercial catalysts for propylene oxidation to acrolein are neat (unsupported) and do not follow this patent's prescribed preparation. This patent also claims that 0.5 to 7 mole % steam is beneficial and recommended. Nowhere in this patent do the authors teach the advantage of anhydrous diluents on product mix nor do they mention flowing heat capacity as a major variable in controlling product selectivity to advantage.

US Patent 4,456,006 teaches a catalyst preparation for the propylene-to-acrolein reaction. It shows that nitrogen diluent presents an improvement over steam diluent when used with this catalyst. It does not teach the heat capacity effect of diluent on product selectivity, nor does it show by-product reductions when using anhydrous diluents.

US Patent 3,717,675 describes a process for recovery of acrylic acid where acrolein is expelled from the aqueous acid collected and returned to the reactors to increase subsequent yields of acrylic acid. This patent mentions the use of inert diluents such as carbon oxides and nitrogen, but does nothing to demonstrate their importance. In fact, it states that it is necessary to add steam to the reaction in order to increase selectivity.

UK Patent 2,068,947 teaches a process for producing methacrolein and methacrylic acid whereby inert anhydrous diluent gases are used, also combined with water vapor to produce a product with a reduced quantity of condensables compared to the typical steam diluent process. The authors fail to recognize the relationship between anhydrous diluents and acetic acid reduction, and they do not address selectivity improvements resultant from use of various anhydrous diluents.

US Patent 4,147,885 describes a recycle process in which steam is an essential ingredient. The object of the patented invention is to recycle steam to the reactors. This is contrary to the techniques of the instant invention, since it has now been found that the reduction or absence of steam is beneficial.

None of the prior art recognizes the use of various inert anhydrous diluents in specific proportions so as to favorably affect the product mix obtained over any of the commonly used catalysts.

As has been indicated above, the basic two-stage process for oxidizing propylene to acrylic acid via acrolein is well known and has been extensively described in the literature. It is also known that wet, overhead gases (noncondensables) from the acrylic acid scrubber can be recycled to the first reactor stage. By this recycling of unreacted propylene and acrolein, it is predictable that an improvement in overall yield is obtained. By use of such a recycle stream, it is also possible to provide a supplemental means of controlling the steam content to the first-stage reactor, as is taught in U.S. Patent No. 4,147,885. In the process of that patent, the steam content of the first-stage feed is required to be 4 to 30% by volume, with all the steam, except that in the starting reactant gas mixture, being provided by the recycle stream. As discussed above, however, the presence of even as little as 4% steam is disadvantageous. This finding has not been addressed, nor even identified, by the prior art.

Co-pending application serial No. 886,562, filed July 17, 1986, describes a process for improving the prior art processes by use of an inert diluent gas having certain heat capacity properties. While that process provides substantial improvements in selectivity and water load, it has been found to have the draw-back of reducing second-stage (acrolein-to-acrylic acid) catalyst efficiency. According to the present invention, there is provided a method for correcting that draw-back.

BRIEF DESCRIPTION OF THE DRAWINGS

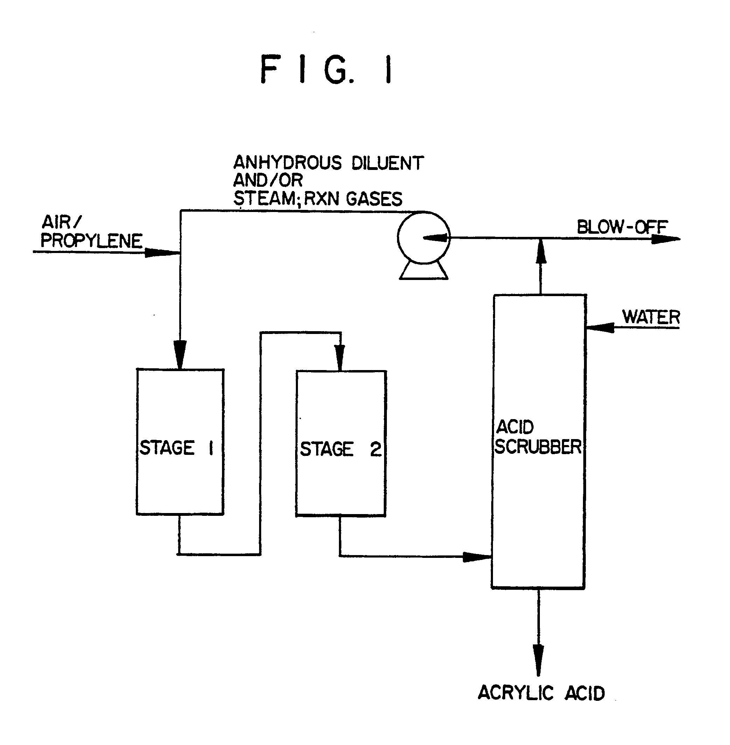

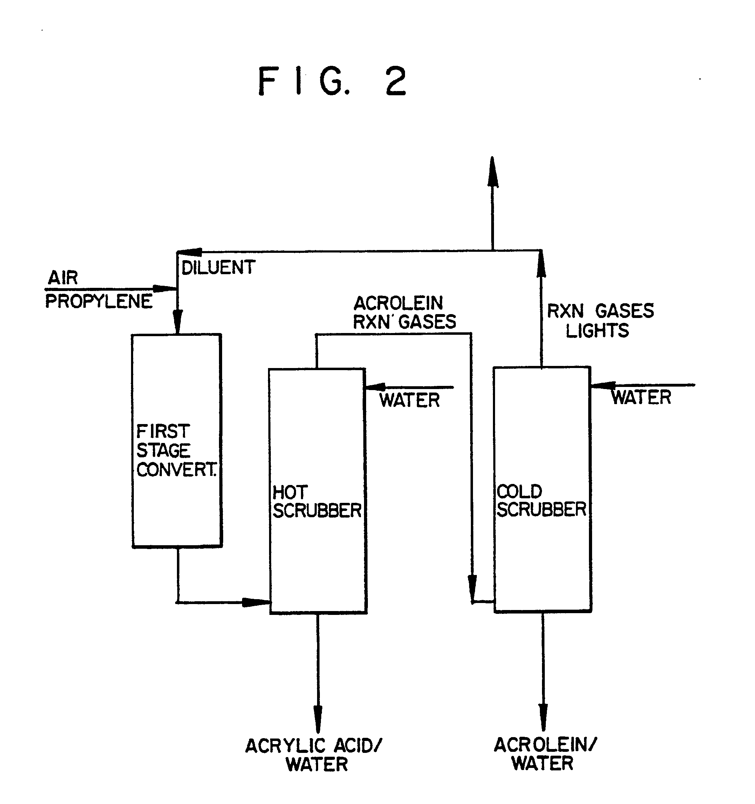

Figures 1 and 2 illustrate the application of recycle streams to acrolein and acrylic acid processes.

## SUMMARY OF THE INVENTION

The present invention embraces two separate but related concepts, namely, the reduction or elimination of steam to reduce the water load on the process, and the improvement of selectivity to maximize the output of desired products. Both of these results are achieved by replacement of some or all of the steam diluent customarily used in prior art processes with an anhydrous diluent (optionally containing a minimal amount of steam) having a heat capacity in a selected range.

According to the present invention, any reduction in second-stage catalyst activity as a result of using the anhydrous diluent of this invention can be compensated for by one, or a combination of, the following: (a) increasing the operating temperature of the second stage catalyst bed by about 5-10°C, (b) increasing the surface area of the second-stage catalyst, and/or (c) adding a limited amount of steam to the second-stage feed (inlet) stream.

In a preferred embodiment, a portion of the non-condensable gases from the process, e.g., the overhead stream from the acrylic acid scrubber, is recycled back to the first-stage reactor feed stream to replace at least some of the steam in that stream.

It will be understood that the process of this invention can be applied not only to a combined propylene-acrolein-acrylic acid process, but also to a separate acrolein-acrylic acid process, or to the acrolein-acrylic acid leg of a propylene-acrylic acid process. Thus, a portion of the product stream from the first-stage propylene-acrolein reactor can be sent to an acrolein recovery process, from which some or all of the non-condensable overhead gases from the acrolein scrubber system can be recycled as diluent to the first and/or second stage of the propylene-acrylic acid process.

## DESCRIPTION OF THE INVENTION

According to the invention, it has been discovered that anhydrous diluent gases can be used to reduce or completely replace steam in the propylene oxidation reaction to efficiently produce acrolein and acrylic acid. (For purposes of this invention, a diluent is any gas which does not react in the reaction stage in which it exists.) Furthermore, when replacing the steam diluent with anhydrous diluents, two major by-products, acetaldehyde and acetic acid, are significantly reduced. These reductions in by-products are especially important since acetaldehyde is difficult to separate from acrolein in recovery operations, and therefore causes an economic penalty in the refining of the product for sale. Likewise, acetic acid and acrylic acid are difficult to separate from each other. To make saleable quality acrylic acid, considerable energy is required to more completely remove the acetic acid. Furthermore, the acetic acid separation step causes acrylic acid recovery losses, and waste disposal costs for disposing of acetic acid are high. The present invention provides a means for reducing acetic acid disposal costs, decreasing separation costs for both acrolein recovery and acrylic acid recovery, and enables existing equipment to enact a better separation, thus saving acrylic acid recovery losses and providing potential for higher quality refined product.

Another key discovery of this invention is that by increasing the flowing heat capacity of the reactant gas mixture, the yield of useful products can be increased significantly. The heat capacity is increased by the introduction of an anhydrous diluent having a relatively high composite heat capacity (as defined herein), comprising one or more gases with relatively high molar heat capacities. Flowing heat capacity is the composite heat capacity of the anhydrous diluent, plus the heat capacity of the reactants, i.e., the composite heat capacity of the total gas stream. However, this does not change appreciably as a result of reaction, since various reaction products have a higher heat capacity than that of the reactants, and some have a lower heat capacity. In general, the flowing heat capacity will not be expected to change by more than about one heat capacity unit as a result of reactions. Thus, the composite heat capacity of the diluent is a dominant variable for process control purposes.

As the flowing heat capacity of the reaction feed gas mixture is increased, yield to acrolein, and acrolein + acrylic acid increase, and the flammable gas range is reduced, enabling higher productivity operations. Simultaneously, the peak temperature in the catalyst bed due to the exothermic heat of reaction is lessened and the heat of reaction that is released is absorbed more efficiently in the bulk gas stream. This, in turn, should increase catalyst life by decreasing thermal stresses within the catalyst pellets' structure, reducing potential carbon build-up within catalyst pores, and by reducing pressure drop, since there will be lower volumetric flow of reactant gas feeds necessary to meet a given production level.

The invention is advantageous for recycling diluent gases and unreacted propylene back to the reactors. The resulting low-steam-containing product streams provide an ample source of noncondensable diluent, so that separation of useful product is simplified. This is particularly advantageous for acrolein recovery, since acrolein, which is more volatile than water, can be effectively separated from the reaction-produced

4

water without loss of diluent. By using anhydrous diluents with higher volatilities compared to acrolein, the present invention permits operation on an acrolein recovery system with recycle of diluent, unreacted propylene, and unrecovered acrolein back to the reactor for further efficiency gains and cost reductions. Such a system using steam diluent is not possible when adapting prior art acrolein recovery equipment and techniques. It also enables implementation of recycle processes in which components such as acetic acid and acrylic acid and other minor, heavy by-products are excluded from the recycle stream. This is significant since the acids and heavy by-products are suspected of adversely affecting catalyst life, and furthermore, it aids in minimizing recycle handling problems, such as compressor corrosion.

The composition of the process feeds must be comprised so that flammable gas mixtures are not formed. According to this invention, the starting reactant gas mixture typically contains up to about 16 g-moles per hour of propylene, preferably up to about 10 g-moles per hour of propylene, per liter of first-stage catalyst ; about 1.1 to about 2.1 moles of molecular oxygen per mole of propylene, and an inert diluent gas which comprises about 40 to about 94% by volume of the feed stream. It is desirable that the mole ratio of composite diluent to propylene be in the range of about 2 to about 32. The diluent gas typically comprises a mixture of nitrogen, carbon dioxide, methane, ethane, propane and steam ; however, any other inert gas can be included. Some other useful inert gases include helium, argon, hydrogen, saturated hydrocarbon gases, $N_2O$, and carbon monoxide. When used, the quantity of steam in this invention should be no more than about 0.4 mole per mole of propylene, preferably 0 to about 0.3 mole per mol of propylene. The inert diluent should be of sufficient quantity to avoid flammable mixtures when combined with the propylene and molecular oxygen. Air or oxygen can be used as the molecular oxygen source, with pure oxygen being preferred. Of course, if air is used, the contained nitrogen acts as a component of the inert diluent.

For each diluent gas mixture there is a relationship which can be determined by experiment and which describes the limiting compositions of oxygen, propylene, and diluent for which flammable mixtures exist. Most commercial applications will be operated in a "fuel-rich" mode, whereby the oxygen content is the limiting factor from a flammability standpoint. The propylene concentrations will be determined by catalyst performance and by commercial cost effectiveness factors.

It is a distinct advantage of this invention that, since diluent gas mixtures with high composite heat capacities have a tendency to broaden the operable range due to shrinkage of the flammable gas envelope, high propylene concentrations are possible. It is theorized that first stage propylene feed concentrations as high as about 30 mole% will be achievable using the method of this invention.

Approximate ranges for feed compositions are defined based on the generalized operating constraints discussed above. First-stage feeds in the following ranges are particularly useful :

Propylene : 0 to 16 g-mole per hour/liter of first-stage catalyst, preferably 0 to 10 g-mole pr hour/liter of first-stage catalyst ;

Oxygen : 1.1 to 2.1 $O_2/C_3H_6$ ratio, such that there is 0 to 33.6 g-mole $O_2$ per hour/liter of first-stage catalyst, preferably 0 to 21 g-mole $O_2$ per hour/liter of first-stage catalyst ;

Diluent : 2.0 to 32 diluent/$C_3H_6$ ratio, preferably 3.5 to 12 diluent/$C_3H_6$ ratio.

The process of the invention is particularly advantageous in that it is not dependent upon any particular catalyst, as is much of the prior art, and will provide its benefits for any catalyst of choice. Any molybdenum, bismuth, iron-based mixed metal oxide catalyst, such as those disclosed in U.S. Patents 3,825,600 ; 3,649,930, and 4,339,355, can be used in the propylene-to-acrolein oxidation reactor. A Mo, V-based mixed metal oxide catalyst, such as described in U.S. patents 3,775,474 ; 3,954,855 ; 3,893,951 ; 4,339,355 can be used effectively in the second stage of the propylene oxidation to acrylic acid (i.e. the acrolein oxidation to acrylic acid reaction).

The general reaction conditions for the first stage are not narrowly critical, and are those known to the art. The first-stage reaction operates at temperatures of 250°C to about 450°C, although temperatures of about 300°C to about 400°C are preferred. The second-stage reaction, according to the prior art, requires temperatures of about 200°C to about 450°C, with a preferred range of about 250°C to about 375°C. According to the present invention, however, this temperature range should, as one option, be raised about 5-10°C.

Operating pressures of about 1 to about 4 atmospheres are typical, although this process improvement will apply for all operating pressures, whether subatmospheric, atmospheric, or superatmospheric. Preferred commercial modes of operation will minimize pressures, but pressures are typically held in the 2 to 3 atm range due to system pressure-drop constraints.

Flow rates can be varied from about 0.5 to about 15 seconds contact time ; however, typical commercial flow provides about 1.5 to about 4.0 seconds contact time. Contact times of about 1.7 to about 3.0 seconds are preferred.

As indicated above, selection of proper heat capacity of the anhydrous diluent gas or gases is critical to the proper performance of the invention. Since the diluent gas stream may comprise a mixture of several individual gases, it is convenient to refer to a composite heat capacity for the total stream. The term "composite heat capacity," as used herein, means the sum of the products of the volumetric fraction of each gas in the diluent gas mixture and its heat capacity. (Heat capacity, as referred to herein, is the ideal gas heat capacity determined at 330°C for purposes of the composite heat capacity definition.) The composite heat capacity for the diluent gas going to the first stage reactor should be at least about 6.5 calories/gram-mole (°C). Below this value, the product selectivity benefits of this invention are minimal. There is no known upper limit on composite heat capacity ; however, it is theorized that above a value of about 40 there may be an unrecoverable heat loss through absorption of reaction heat into the process stream, which would result in an economic penalty. In addition, there could be a problem with increased after-burning at the exit of the first-stage reactor. It is preferred that the composite heat capacity be maintained in the range of about 8 to 20, and most preferably about 10 to 17.

The flowing heat capacity of the second-stage reactor feed gases is determined predominantly by the choice of anhydrous diluent gas fed to the first-stage reactor. The first-stage product mix has only a minor influence on the second-stage feed flowing heat capacity, since the products account for only about ten to twenty percent of the total stream volume. For example, a typical operation with 7% propylene and 13% oxygen produces water plus acrolein, acrylic acid, acetaldehyde, acetic acid, and carbon oxide. The average heat capacity of the feed propylene and oxygen is very nearly the same as the average heat capacity of the resulting products (approximately 0.65 cal/g-mole (0°C) more for products versus reactants).

The anhydrous diluent gas of this invention can be a single gas or a multi-component mixture of gases, provided that certain criteria are observed. Each gas must be inert to the oxidation reactions of the process, and each gas must be noncondensable and readily separable from the reaction products.

Since each plant installation will have specific constraints that affect the energy usage for the entire plant, attention must be paid to the impact on the plant energy balance for use of particular diluents which alter the current heat recovery schemes. For example, a high heat capacity diluent will retain more of the heat evolved through reaction, whereas now the bath temperature is relied on more to remove and recover the heat of reaction. High heat capacity diluents will require more attention to recovering heat after the reaction. Furthermore, if process off-gas is disposed of via combustion, the recovery of heat will be affected by a major change in diluents.

In addition, one should avoid catalyst poisons, e.g., sulfur dioxide, and gases that react to unwanted by-products, as would $C_4$-unsaturated compounds, or $NH_3$, which produces acrylonitrile.

It is another advantage of this invention that the steam component typically contained in the feed to the first stage can be minimized, even eliminated. While there is controversy among those skilled in the art as to the precise function of steam, e.g., whether it is truly an inert diluent or whether it somehow participates in the oxidation of propylene and acrolein, it is accepted practice in the art that a significant concentration of steam is required in order to successfully operate the first-stage and second-stage reactions. Contrary to this holding of the art, it is a surprising discovery of the present invention that steam is desirably minimized and can be eliminated entirely. This is accomplished by substituting for the steam the inert gas diluent of selected composite heat capacity of this invention. Accordingly, it has been found that the steam content of the feed gas can be essentially zero. While not preferred, the steam content of the feed gas can range as high as about 3% by volume of the feed gas. It is preferred that the steam content of the feed stream be kept below about 2%, more preferably below about 1%, by volume.

As indicated above, it has been observed that in the second stage (acrolein-to-acrylic acid reaction step), the replacement of a substantial amount of steam with anhydrous diluent can reduce the activity of the second-stage catalyst. According to the present invention, it has been found that second-stage catalyst activity can be restored by increasing the operating temperature of the second stage by about 5-10°C above that required if the second stage inlet stream contained about 45 mole percent steam. According to the present invention, it has also been found that second-stage catalyst activity can be restored by introducing steam to the second stage, such that the steam content is preferably no more than about 45 mole percent of the inlet stream to the second stage. When introducing steam to the second-stage reactor, the steam content of the inlet stream should be adjusted to no more than about 45 mole percent of the inert gas components of that stream. Most preferably, the steam content will be no more than about 20 mole percent of the inlet gas stream. Additionally, according to the present invention it has been found that a reduction in second-stage catalyst activity can be corrected by increasing the available surface area of the second-stage catalyst sufficiently to compensate for the observed loss of catalyst efficiency. Such increase in catalyst surface area can be accomplished by utilizing smaller catalyst pellet size, or more efficient shape, or by increasing the length of the catalyst bed.

While not an absolute requirement of this invention, it is highly preferred that the inert diluent gas used be, at least in part, an essentially anhydrous recycle stream from within the process. Preferably, this will comprise a portion of the noncondensable, overhead gas mixture from the acrolein or acrylic acid recovery scrubber train which removes water and acrylic acid from the product mixture. In particular, the use of low-boiling, anhydrous diluents makes recycle from an acrolein recovery process possible. Besides providing a beneficial system which allows recovery of current acrolein separation efficiency losses and reuse of unconverted propylene, recycling process gases from an acrolein recovery process is desirable and advantageous to recycling process gases from an acrylic acid recovery process as described in the prior art.

In order to minimize the water vapor carried overhead from these scrubbers, they should be operated within the ranges of conditions shown in Table I.

## Table I

| | ACRYLIC ACID SCRUBBERS: (Acrolein Recovery System Or Acrylic Acid Recovery System | ACROLEIN SCRUBBER: (Acrolein Recovery System) |
|---|---|---|
| BASE TEMP. (°C) | <95,preferably: <80 | <45, pref.: 15 to 35 |
| HEAD TEMP. (°C) | <80, pref.: <70 most. pref.: <60 | <40, pref.: 10 to 30 |
| PRESSURE (ATM) | <3, pref. 1. to 2. | <3, pref.: 1. to 2. |
| SCRUBBING MEDIUM FLOW (Volume) BOTTOM PRODUCT STREAM FLOW (Volume) | <1/1 pref.: <1/2. | |
| SCRUBBING MEDIUM FLOW (Weight) ACROLEIN BOTTOMS FLOW (Weight) | | <80/1 pref.: <30/1 |

Under most operating conditions, it will be necessary to take off a purge stream, the size and location of which will be determined by the specific process being used. If pure oxygen is used as the source of oxygen, the purge can be relatively small. If air is used as the oxygen source, there will be a build-up of inerts, e.g., nitrogen, so that a substantial purge will be required, and will be controlled to maintain the desired composite heat capacity. The use of pure oxygen (i.e., oxygen not burdened with substantial concentrations of inert gases) permits maximization of diluents with a heat capacity higher than that of nitrogen. This permits minimization of the purge, which in turn, makes feasible the use of high heat capacity gases, such as propane, which might be too expensive for use as diluents if they were to be substantially lost through purging.

In the examples below, various diluents were examined in a pilot-scale reactor system consisting of two single tubular reaction vessels of typical commercial reactor tube dimensions. The first reactor tube contained a commercial catalyst comprising molybdenum, bismuth, iron and several promoter metals typical of first-stage catalyst, as described above. The second reactor tube was filled with a commercial second-stage catalyst, similar to those described previously, and was connected in series with the first. The gaseous reaction products were sampled and separated into condensable and noncondensable portions. Each phase sample was measured and analyzed by gas chromatograph. The resultant measurements were used to calculate reaction yields and propylene conversions. These sampling procedures were accomplished for both first-stage product and second-stage product, so that process performance for acrolein production and process performance for acrylic acid production were both determined.

The experiments were set up in a statistical design, and the same statistical design was used for several diluents. These diluents included nitrogen, carbon dioxide, methane, propane and steam.

Additional investigations included a statistically designed set of experiments in which the composite heat capacity of the diluent gases was varied systematically in a series of recycle runs in which fresh air and propylene were fed to the reactors along with a diluent gas stream obtained by recycling a portion of the uncondensable product stream from the second-stage reactor.

In all the designed experiment sets, the prototype reaction feed concentrations of 7.0 mole% propylene, 60.2 mole% air, and 32.6 mole% diluent were used. The diluent consisted of steam plus an anhydrous diluent additive. The steam-to-anhydrous diluent additive ratio was varied as discussed in the design below.

The experiments were run in a $2^3$- factorial design with four center points. The independent variables were first-stage temperature, space velocity, and steam feed concentration. The propylene feed concentration (7 mole%) and air-to-propylene feed concentration ratio (8.6) were fixed, as were the system pressure and second-stage operating conditions. The major objective of the experimental set-up was to describe the first-stage (acrolein) catalyst performance with various concentrations of anhydrous diluent and steam. The designed set of experiments is outlined below.

| | + | Center Point | − |
|---|---|---|---|
| space velocity (hr$^{-1}$) | 2000 | 1600 | 1200 |
| temperature (°C) | 340 | 330 | 320 |
| steam conc. (mole %) | 30 | 20 | 10 |
| (inferred anhydrous diluent conc. (mole %)) | 2.6 | 12.6 | 22.6 |

By the term "inferred" is meant the diluent gas added to the air, propylene, and steam mixture. This number does not take into account the nitrogen diluent inherent in the air feed.

In addition to the basic designed set of experiments, two 0% steam runs were run at outlying points of the experimental space. A composition profile run was made at 0% steam and 1600 hr$_{-1}$ space velocity. Several additional tests were made to verify hotspot temperature observations. Linear regression models that gave the best-line-fit with minimal residual variance were developed. The t-ratio for each contributing independent variable had at least a 95% confidence limit on its significance, based on standard statistics calculations. Most variables had a 99% confidence limit if included in the model equations. In general, the equations provided a very good fit to the data.

The terms "conversion," "yield," "selectivity," "space velocity," and "contact time" are defined as follows:

$$\text{conversion \%} = \frac{\text{moles propylene converted}}{\text{moles propylene fed}} \times 100$$

$$\text{yield (mole\%)} = \frac{\text{moles product produced}}{\text{moles propylene fed}} \times \frac{\text{number carbon atoms in product}}{3} \times 100$$

$$\text{selectivity (mole\%) b} = \frac{\text{moles product produced}}{\text{moles propylene converted}} \times \frac{\text{number carbon atoms in product}}{3} \times 100$$

$$\text{space velocity (hr}^{-1}) = \frac{\text{gas volumetric flowrate (1/hr)}^*}{\text{volume of reactor catalyst bed (1)}}$$

$$\text{contact time (seconds)} = \frac{3600}{\text{space velocity}}$$

* Flow adjusted to standard temperature and pressure (i.e., 0°C and 1 atm).

## EXAMPLES

The examples which follow illustrate the invention, but are not intended to limit it in any way. In these examples, all concentrations are in mole percent.

### Example 1

The experimental set-up for the pilot plant reactor tubes was as described above. It consisted of two like tubular reactors, each consisting of one tube filled with an appropriate catalyst, as described above. A jacket surrounding each tube was filled with a heat transfer fluid which circulated to remove heat of reaction. Thermocouple and sample taps were provided along the length of each reactor and at the bottom of each reactor. Gas feeds were metered into the first reactor using mass flowmeters. The first-stage effluent was then conducted directly into the second-stage reactor. The condensable portion of the second-stage effluent was recovered as a liquid tails stream from a water-based scrubber. Non-condensable gases were conducted out the top of the scrubber, and could be returned, if desired, to the reactors to supply additional diluent gas. The system outlet pressure was controlled at 7 psig in order to control reactor feed pressures on the system. Propylene feed concentration was set at 7.0% and air feed concentration was set at 60.2%. The additional (to nitrogen in the air feed) feed gas diluent contained 2.6% nitrogen and 30% steam. (Also present was about 0.2% inert impurities in the propylene.) The system outlet pressure was set at 7 psig, and reactor temperature was adjusted to 320°C. The results are shown in Table 1.

### Example 2

Example 1 was repeated, but the additional diluent feed contained 12.6% nitrogen and 20% steam. See Table 1 for results.

### Example 3

Example 1 was repeated, but the diluent feed was 22.6% nitrogen and 10% steam.

### Example 4

Example 1 was repeated, but with 32.6% nitrogen and 0% steam as the diluent gas. Temperature was adjusted to give a first-stage propylene conversion of 94.5%.

### Examples 5 through 8

The conditions of Examples 1 through 4 were repeated with methane instead of nitrogen in the diluent gas, the remainder being steam.

These first eight examples of the first-stage catalyst performance clearly demonstrate that as the steam feed concentration decreases (descending order for Examples 1 through 4 and 5 through 8), that the total acetaldehyde + acetic acid yield also decreases. This decrease is then directly translated to the second-stage reaction in the overall determination of acetic acid yield. Furthermore, in directly comparing Examples 1 through 4 to Examples 5 through 8, it is evident that acrolein + acrylic acid yield is significantly higher for the methane (higher heat capacity), compared to nitrogen, diluent. Again, these yields translate directly to the overall two-stage acrylic acid yield.

Over the range of these experiments, each percentage point of steam in the feed to the first stage provides a 2.6% increase in the acetaldehyde-plus-acetic acid yield above the 0% steam level. This is shown in the following relationship :

Acetaldehyde + Acetic Acid Yields =
1.7535 + 0.0304 (mole % steam - 20)

## TABLE I
### FIRST-STAGE PERFORMANCE

| | Mole % $CH_4$ | Mole % $N_2$ | Mole % $H_2O$ | (f) $C_p$ | $SV^{(a)}$ $(hr^{-1})$ | (°C) $I_1$[b] | (°C) $I(^c)_{HOT}$ | % $C_3$ Conv.[d] | Acrolein | Acrylic Acid | HAc + HOAc[e] |
|---|---|---|---|---|---|---|---|---|---|---|---|
| | | | | | | | | | | Mole % Yields | |
| Example 1 | – | 2.6 | 29.5 | 7.9 | 1985.2 | 320.1 | 388.9 | 93.3 | 77.1 | 10.4 | 1.95 |
| Example 2 | – | 12.6 | 19.92 | 7.73 | 1689.6 | 329.2 | 393.4 | 96.99 | 77.97 | 13.35 | 1.84 |
| Example 3 | – | 22.6 | 10.16 | 7.55 | 1994.2 | 320.4 | 398.3 | 94.85 | 76.9 | 11.9 | 1.57 |
| Example 4 | – | 32.6 | 0 | 7.37 | 1585.1 | 314.5 | 397.3 | 94.48 | 81.97 | 6.97 | 1.01 |
| Example 5 | 2.6 | – | 29.51 | 8.07 | 1931.4 | 320.8 | 389.0 | 95.06 | 80.17 | 9.67 | 1.85 |
| Example 6 | 12.6 | – | 20.55 | 8.56 | 1611.8 | 330.3 | 394.2 | 97.32 | 79.5 | 12.3 | 1.66 |
| Example 7 | 22.6 | – | 10.74 | 9.05 | 1989.3 | 319.9 | 400.1 | 95.92 | 79.32 | 10.98 | 1.47 |
| Example 8 | 32.6 | – | 0 | 9.54 | 1562.2 | 324.4 | 387.8 | 95.7 | 80.4 | 10.8 | 0.68 |

(a) Space Velocity
(b) First-stage reactor bath temperature
(c) First-stage reactor peak catalyst temperature
(d) Propylene Conversion
(e) Acetic Acid + Acetaldehyde
(f) Composite heat capacity, cal/g mole–°C, based on ideal gas heat capacities at 330°C.

### Example 9

The experimental set-up for Examples 1 through 8 was duplicated ; however, no additional anhydrous diluent was used, i.e., the feeds consisted of 60.2% air, 7.0% propylene, and 32.6% steam. This is considered the conventional composition for use as a comparison in Examples 10 through 14. Results of Examples 9 through 14 are displayed in Table II.

### Example 10

The experimental set-up used in Examples 1 through 9 was repeated. Propylene feed concentration was set at 7.0% with air as the molecular oxygen-containing gas, in the amount of 60.2% of the reactor feed stream. The remaining feed was comprised of diluent gas made up of propane and nitrogen. The quantities of nitrogen and propane were adjusted so that the combined gas mixture had the same molar heat capacity as found with methane alone. This amounted to 6.4% propane and 26.2% nitrogen in the reactor feed gas stream. System outlet pressure was controlled at 7 psig, and reactor temperature was maintained at 330°C.

### Example 11

Example 10 conditions were duplicated with the exception that the diluent gas was comprised so that it had the same average molar heat capacity as steam. Propane diluent concentration was 2.0% and nitrogen diluent concentration was 30.6% of the reactant feed mixture. The results for Examples 10 and 11 are tabulated in Table II.

### Example 12

The conditions of Example 11 were repeated with a propane concentration of 10.8% and a nitrogen concentration of 21.8%.

### Example 13

The conditions of Example 11 were repeated with a propane concentration of 23.0% and a nitrogen concentration of 9.6%.

### Example 14

In this example, the propylene feed and air feed flow rates were set identical to the conditions used in Examples 10 through 13. In this case, the volumetric flow of diluent was lowered, but the composite heat capacity of this diluent was the same as that of diluent used in Example 12. This was done by running the following feed conditions : 1380 hr$_{-1}$ space velocity, 8.33% propylene, 69.8% air, 14.6% nitrogen, and 7.26% propane. The results are given in Table II.

By comparing the acrolein + acrylic acid yield in Examples 9 through 13, it is clear that higher heat capacity provides higher efficiency to useful products. Furthermore, in comparing Examples 10 through 13 to Example 9, it is seen that the absence of steam diluent gives a dramatic reduction in acetaldehyde + acetic acid yields. Example 14 shows that by using diluents with high composite heat capacity, the process can be run using lower volumetric flow rates of diluent while maintaining high yields of useful products.

Over the range of these experiments, with composite heat capacities in the 7 to 14.1 range, the following relationships represent the expected trends in useful product yields :

Acrolein yield = 1.224(CCP) + 69.3084

Acrolein & Acrylic acid yield = 0.756(CCP) + 83.7744

where CCP = diluent composite heat capacity, as defined above.

## TABLE II
### FIRST-STAGE PERFORMANCE

| Diluent | (Cql/gmol°C) Composite Cp | SV | $I_1$ | % $C_3$ Conv. | % Yld. to: $CO_2$ | CO | HAc | ACR | HOAc | AA | AA + ACR |
|---|---|---|---|---|---|---|---|---|---|---|---|
| Steam (Ex. 9) | 7.95 | 1600 | 325 | 95.6 | 2.3 | 1.5 | 1.20 | 77.9 | 0.6 | 11.5 | 89.4 |
| $C_3H_8/N_2$ (Ex.11) | 7.95 | 1600 | 320 | 95.02 | 2.89 | 1.61 | .63 | 79.57 | .31 | 9.75 | 89.31 |
| $C_3H_8/N_2$ (Ex.10) | 9.25 | 1600 | 330 | 96.61 | 3.39 | 2.06 | .63 | 76.3 | .43 | 13.6 | 89.9 |
| $C_3H_8/N_2$ (Ex. 12) | 10.55 | 1600 | 320 | 96.65 | 1.84 | 1.25 | 0.71 | 82.91 | .29 | 10.4 | 93.29 |
| $C_3H_8/N_2$ (Ex. 13) | 14.13 | 1600 | 320 | 98.31 | 1.85 | .967 | 0.72 | 86.65 | .32 | 7.31 | 93.96 |
| $C_3H_8/N_2$ (Ex. 14) | 9.59 | 1380 | 320 | 96.66 | 3.15 | 1.72 | 0.73 | 79.8 | .36 | 10.7 | 90.5 |

12

## Recycle Applications

Recycling of process streams is well known in the chemical process arts, and is usually implemented to improve reaction efficiencies and process economics. More specifically, recycling of product or a portion of a product stream enables efficient use of feed material not reacted in a single pass or reuse of feed material which is costly to make up in the reactor feed stream. Use of anhydrous diluents has a particularly advantageous effect on the operability of recycle. It enables using a recycle stream which has less acid, thus increasing compressor operability. Furthermore, prior art recycle processes require more elaborate sampling mechanisms in order to reliably measure recycled oxygen concentrations. The control of oxygen is essential to safe operation of these recycle processes due to concerns over flammable gas mixtures. The anhydrous streams of this invention, however, provide for reliable and accurate monitoring of oxygen, thereby increasing the recycle process reliability and operability as well as safety.

. Furthermore, an anhydrous diluent process allows simple, efficient recovery and recycle of acrolein in an acrolein production unit. Figures 1 and 2 illustrate the application of recycle to the acrolein and acrylic acid processes.

## Example 15 (recycle)

The product gas from the second-stage reactor was condensed to remove all condensable components. A portion of uncondensed components of this product stream containing nitrogen, carbon dioxide, carbon monoxide, oxygen, and propylene was directed to a compressor and compressed up to approximately 30 psig for feed to the reactors. The quantities of oxygen and propylene coming over in this recycle stream were calculated, and make-up propylene and air were added so that the reactor feed stream contained 7.0% propylene and 12.6% oxygen. The temperature of the first-stage reactor was controlled so that propylene conversion was 95%. The second-stage temperature was likewise controlled so that acrolein conversion was more than 99%. The first-stage acrolein yield was measured at 78% and the first-stage acrylic acid yield measured at 12.2%. The overall (two-stage) yield to acrylic acid averaged 85%.

## Claims

1. A process for producing acrylic acid by a two-stage catalytic oxidation of propylene, wherein the first stage produces primarily acrolein and the second stage produces primarily acrylic acid by oxidation of acrolein, said process utilizing one or more recycle streams to either or both stages, both stages operating on feed streams containing oxygen and an inert diluent gas, the improvement comprising (a) utilizing as the diluent gas to the first stage a gas mixture comprising about 0 to about 0.4 mole of steam per mole of propylene, the remainder of the diluent gas comprising one or more inert gases such that the diluent gas has a composite heat capacity of at least about 6.5 calories/gram-mole (°C), and (b) utilizing as the diluent gas to the second stage a gas comprising one or more inert gases having a composite heat capacity of at least about 6.5 calories/gram-mole (°C), one of which inert gases is preferably steam.

2. A process for producing acrylic acid of claim 1, characterized in increasing the operating temperature of the second stage by about 5-10°C above that required if steam were present in the inlet stream to the second stage at a concentration of about 45 mole percent of the inlet stream to the second stage.

3. A process for producing acrylic acid of one or both of the claims 1-2, charcterized in increasing the available surface area of the second-stage catalyst sufficiently to compensate for any loss of catalyst efficiency caused by reduction or elimination of steam in the inlet stream to the second stage.

4. A process of one or more of the claims 1-3 wherein the composite heat capacity of the inert diluent gas to the first stage is about 6.5 to about 40, e.g. about 8 to about 20, preferably about 10 to about 17.

5. A process of one or more of the claims 1-4 wherein the steam content of the diluent gas to the first stage is less than about 0.4 mole, e.g. less than about 0.3 mole per mole of propylene.

6. A process of one or more of the claims 1-5 wherein the steam content of the inert diluent gas to the second stage is no more than about 45 mole percent of the inlet stream to the second stage.

7. A process of one or more of the claims 1-6 wherein the composite heat capacity of the inert diluent gas to the second stage is about 6.5 to about 40, e.g. about 8 to about 20, preferably about 10 to about 17.

8. A process of one or more of the claims 1-7 wherein the oxygen is from a pure oxygen source.

9. A process of one or more of the claims 1-8 wherein the diluent gas comprises a recycled process stream from an acrolein recovery operation or an acrylic acid recovery operation.

## Ansprüche

1. Verfahren zur Herstellung von Acrylsäure nach einer katalytischen Zwei-Stufen-Oxidation von Propylen, worin die erste Stufe hauptsächlich Acrolein bildet und die zweite Stufe hauptsächlich Acrylsäure durch Oxidation von Acrolein bildet, wobei das Verfahren ein oder mehrere Rückführungsströme zu einer oder beiden Stufen verwendet, beide Stufen mit Beschickungsströmen arbeiten, die Sauerstoff und ein inertes Verdünnungsgas enthalten, wobei die Verbesserung umfaßt : (a) Verwendung einer etwa 0 bis etwa 0,4 Mol Wasserdampf pro Mol Propylen umfassenden Gasmischung als Verdünnungsgas zur ersten Stufe, wobei der Rest des Verdünungsgases ein oder mehrere inerte Gase umfaßt, so daß das Verdünnungsgas eine zusammengesetzte Wärmekapazität von mindestens etwa 6,5 cal/gramm-Mol (°C) hat, und (b) Verwendung eines Gases als Verdünnungsgas zur zweiten Stufe, das ein oder mehrere inerte Gase mit einer zusammengesetzten Wärmekapazität von mindestens etwa 6,5 cal/gramm-Mol (°C) umfaßt, wobei eines der inerten Gase vorzugsweise Wasserdampf ist.

2. Verfahren zur Herstellung von Acrylsäure gemäß Anspruch 1, dadurch gekennzeichnet, daß die Betriebstemperatur der zweiten Stufe um etwa 5 bis 10°C über diejenige erhöht wird, die notwendig wäre, wenn Wasserdampf im Eintrittsstrom zur zweiten Stufe in einer Konzentration von etwa 45 Mol-% des Eintrittsstromes zur zweiten Stufe anwesend wäre.

3. Verfahren zur Herstellung von Acrylsäure nach einem oder beiden der Ansprüche 1 bis 2, dadurch gekennzeichnet, daß die verfügbare Oberfläche des Katalysators der zweiten Stufe ausreichend erhöht wird, um jeglichen durch Verringerung oder Eliminierung von Wasserdampf im Eintrittsstrom zur zweiten Stufe verursachten Wirksamkeitsverlust das Katalysators zu kompensieren.

4. Verfahren nach einem oder mehreren der Ansprüche 1 bis 3, worin die zusammengesetzte Wärmekapazität des inerten Verdünnungsgases zur ersten Stufe etwa 6,5 bis etwa 40, z.B. etwa 8 bis etwa 20, vorzugsweise etwa 10 bis etwa 17, beträgt.

5. Verfahren nach einem oder mehreren der Ansprüche 1 bis 4, worin der Wasserdampfgehalt des Verdünnungsgases zur ersten Stufe geringer als etwa 0,4 Mol, z.B. geringer als etwa 0,3 Mol, pro Mol Propylen ist.

6. Verfahren nach einem oder mehreren der Ansprüche 1 bis 5, worin der Wasserdampfgehalt des inerten Verdünnungsgases zur zweiten Stufe nicht mehr als etwa 45 Mol-% des Eintrittsstromes zur zweiten Stufe beträgt.

7. Verfahren nach einem oder mehreren der Ansprüche 1 bis 6, worin die zusammengesetzte Wärmekapazität des inerten Verdünnungsgases zur zweiten Stufe etwa 6,5 bis etwa 40, z.B. etwa 8 bis etwa 20, vorzugsweise etwa 10 bis etwa 17, beträgt.

8. Verfahren nach einem oder mehreren der Ansprüche 1 bis 7, worin der Sauerstoff aus einer Quelle reinen Sauerstoffs stammt.

9. Verfahren nach einem oder mehreren der Ansprüche 1 bis 8, worin das Verdünnungsgas einen zurückgeführten Verfahrensstrom aus einem Acroleingewinnungs-Vorgang oder einem Acrylsäuregewinnungs-Vorgang umfaßt.

## Revendications

1. Procédé de production d'acide acrylique par oxydation catalytique du propylène en deux étapes, dans lequel la première étape produit principalement de l'acroléine et la seconde étape produit principalement de l'acide acrylique par oxydation de l'acroléine, ledit procédé utilisant un ou plusieurs courants recyclés dans l'une ou l'autre des étapes ou dans les deux, les deux étapes opérant sur des courants d'alimentation contenant de l'oxygène et un diluant gazeux inerte, le perfectionnement consistant (a) à utiliser comme diluant gazeux introduit dans la première étape un mélange gazeux comprenant environ 0 à environ 0,4 mole de vapeur d'eau par mole de propylène, le reste du diluant gazeux comprenant un ou plusieurs gaz inertes de manière que le diluant gazeux ait une capacité calorifique globale d'au moins environ 6,5 calories/molécule-gramme (°C) et (b) à utiliser comme diluant gazeux introduit dans la seconde étape un gaz comprenant un ou plusieurs des gaz inertes ayant une capacité calorifique globale d'au moins environ 6,5 calories/molécule-gramme (°C), l'un de ces gaz inertes étant de préférence la vapeur d'eau.

2. Procédé de production d'acide acrylique suivant la revendication 1, caractérisé par l'élévation de la température de travail de la seconde étape d'environ 5 à 10°C au-dessus de la température nécessaire si de la vapeur d'eau est présente dans le courant admis à la seconde étape à une concentration d'environ 45 moles% du courant admis à la seconde étape.

3. Procédé de production d'acide acrylique suivant l'une des revendications 1 et 2 ou ces deux revendications, caractérisé par l'élévation de la surface spécifique disponible du catalyseur de la seconde étape dans une mesure suffisante pour compenser toute perte de rendement du catalyseur provoquée par la réduction de la quantité de vapeur d'eau ou par son élimination dans le courant admis à la seconde étape.

4. Procédé suivant une ou plusieurs des revendications 1 à 3, dans lequel la capacité calorifique globale du diluant gazeux inerte admis à la première étape est d'environ 6,5 à environ 40, par exemple d'environ 8 à environ 20, de préférence d'environ 10 à environ 17.

5. Procédé suivant une ou plusieurs des revendications 1 à 4, dans lequel la teneur en vapeur d'eau du diluant gazeux admis à la première étape est inférieure à environ 0,4 mole, par exemple inférieure à environ 0,3 mole par mole de propylène.

6. Procédé suivant une ou plusieurs des revendications 1 à 5, dans lequel la teneur en vapeur d'eau du diluant gazeux inerte admis à la seconde étape ne dépasse pas environ 45 moles% du courant admis à la seconde étape.

7. Procédé suivant une ou plusieurs des revendications 1 à 6, dans lequel la capacité calorifique globale du diluant gazeux inerte admis à la seconde étape est d'environ 6,5 à environ 40, par exemple d'environ 8 à environ 20, de préférence d'environ 10 à environ 17.

8. Procédé suivant une ou plusieurs des revendications 1 à 7, dans lequel l'oxygène provient d'une source d'oxygène pur.

9. Procédé suivant une ou plusieurs des revendications 1 à 8, dans lequel le diluant gazeux comprend un courant de fabrication recyclé provenant d'une opération de récupération d'acroléine ou d'une opération de séparation d'acide acrylique.

# F I G. I

ANHYDROUS DILUENT
AND/OR
STEAM; RXN GASES

AIR/
PROPYLENE

BLOW-OFF

WATER

STAGE I

STAGE 2

ACID
SCRUBBER

ACRYLIC ACID

# F I G. 2